# EUROPÄISCHE PATENTSCHRIFT

(11) **EP 2 776 490 B1**
(45) Veröffentlichungstag und Bekanntmachung des Hinweises auf die Patenterteilung: **28.02.2018**
(21) Anmeldenummer: 12784583.2
(22) Anmeldetag: 08.11.2012
(51) Int. Cl.: C08G 59/50, C08G 59/18

(54) **HÄRTER FÜR EPOXIDHARZ-BESCHICHTUNGEN**
HARDENERS FOR EPOXY RESIN COATINGS
MATÉRIAU D'AUTO-RÉTABLISSEMENT ET PROCÉDÉ DE PRÉPARATION ASSOCIÉ

(30) Priorität: 10.11.2011 EP 11188693
(43) Veröffentlichungstag der Anmeldung: 17.09.2014
(73) Patentinhaber: Sika Technology AG, 6340 Baar (CH)
(72) Erfinder: BURCKHARDT, Urs, CH-8049 Zürich (CH); KASEMI, Edis, CH-8046 Zürich (CH)
(74) Vertreter: Sika Patent Attorneys
(86) Internationale Anmeldenummer: PCT/EP2012/072193
(87) Internationale Veröffentlichungsnummer: WO 2013/068502

(56) Entgegenhaltungen:
- US-A- 3 574 221
- US-A- 4 120 913
- US-A- 4 978 792
- US-A1- 2009 163 676

## Beschreibung

### Technisches Gebiet

Die Erfindung betrifft das Gebiet der Amine als Härter für Epoxidharze, sowie Amine enthaltende Epoxidharz-Zusammensetzungen und ihre Verwendung, insbesondere als Beschichtung wie in den vorliegenden Ansprüchen beschrieben.

### Stand der Technik

Epoxidharz-Zusammensetzungen sollen eine Reihe von Eigenschaften aufweisen, um als Beschichtung von hoher Qualität verwendbar zu sein. Einerseits sollen sie eine niedrige Viskosität aufweisen, damit sie bei Umgebungstemperatur gut verarbeitbar und selbstverlaufend sind, und sie sollen ohne sogenannte Blushing-Effekte schnell aushärten, auch bei feucht-kalten Bedingungen. Als "Blushing" werden Mängel bei der Aushärtung wie Trübungen, Flecken und raue oder klebrige Oberfläche bezeichnet, verursacht durch Salzbildung von Aminen mit Kohlendioxid (CO₂) aus der Luft, wobei eine hohe Luftfeuchtigkeit und tiefe Temperaturen das Auftreten von Blushing-Effekten begünstigen. Im ausgehärteten Zustand soll die Epoxidharz-Beschichtung eine ebenmässige Oberfläche ohne Trübungen, Flecken oder Krater aufweisen, und sie soll eine hohe Härte und gute Beständigkeit besitzen. Um diese Eigenschaften zu erreichen, werden in Epoxidharz-Beschichtungen nach dem Stand der Technik üblicherweise Verdünner eingesetzt. Solche Verdünner, wie Benzylalkohol oder Phenole, werden bei der Aushärtung nicht in die Harzmatrix eingebaut. Immer wichtiger wird heutzutage aber die Forderung nach emissionsarmen Systemen, die nach der Aushärtung einen geringen Gehalt von durch Verdampfungs- oder Diffusionsprozesse freisetzbaren Substanzen aufweisen. Für emissionsarme Systeme können nicht einbaubare Verdünner deshalb nur in sehr geringer Menge oder gar nicht verwendet werden.
US 2009/0163676 beschreibt Härter-Zusammensetzungen enthaltend mindestens ein benzyliertes Polyalkylenpolyamin und mindestens ein weiteres Amin. Ohne Adduktierung mit Epoxiden härten diese Härter mit Epoxidharzen vor allem in der Kälte nur langsam aus. Eine teilweise Adduktierung an Epoxide bewirkt zwar eine schnellere Aushärtung, die Viskosität der Härter wird dabei aber stark erhöht. Das Beispiel 9 in US-A-3574221 offenbart ein aromatisches tertiäres Amin, das in einer pharmazeutischen Zusammensetzung verwendet wird.

### Darstellung der Erfindung

Aufgabe der vorliegenden Erfindung ist es daher, einen Härter für Epoxidharze zur Verfügung zu stellen, der niedrigviskos ist, zusammen mit Epoxidharzen gut verarbeitbar ist und mit diesen auch bei feucht-kalten Bedingungen schnell und ohne Blushing-Effekte zu Beschichtungen von hoher Härte und Beständigkeit aushärten kann.
Überraschenderweise wurde gefunden, dass Härter nach nach den vorliegenden Ansprüchen enthaltend Amine mit mindestens einer Aminogruppe der Formel (I) diese Aufgabe lösen. Die Härter nach Anspruch 1 sind geruchsarm und mit Epoxidharzen gut verträglich. Ihre Viskosität ist überraschend niedrig, insbesondere im Vergleich zu ähnlichen Aminen, welche anstelle der tertiären Aminogruppe eine andere, den Phenylring aktivierende Gruppe, zum Beispiel eine Hydroxylgruppe (Phenolgruppe), aufweisen. Die Härter weisen eine überraschend geringe Farbtönung auf, trotz der leichten Verfärbbarkeit der Ausgangsverbindungen. Mit Epoxidharzen härten sie überraschend schnell aus, insbesondere auch bei feucht-kalten Bedingungen, wobei Beschichtungen mit einer erstaunlich geringen Verfärbungsneigung entstehen.
Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Härter, wie in den vorliegenden Ansprüchen beschrieben, geeignet zum Aushärten von Epoxidharzen, enthaltend mindestens ein Amin mit mindestens einer Aminogruppe der Formel (I), 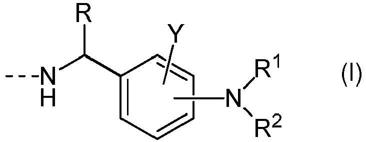 wobei
R für ein Wasserstoffatom oder eine Alkylgruppe mit 1 bis 12C-Atomen steht, bevorzugt für ein Wasserstoffatom oder eine Methylgruppe, insbesondere für ein Wasserstoffatom;
R¹ und R² jeweils unabhängig voneinander für eine Alkyl- oder Arylalkylgruppe mit 1 bis 12 C-Atomen stehen, oder zusammen für eine Alkylengruppe mit 4 bis 5 C-Atomen stehen, bevorzugt für mindestens eine Alkylgruppe mit 1 bis 4 C-Atomen, insbesondere für eine Methylgruppe; und
Y für ein Wasserstoffatom oder für eine Alkyl- oder Alkoxygruppe mit 1 bis 12 C-Atomen steht, bevorzugt für ein Wasserstoffatom oder eine Methylgruppe, insbesondere für ein Wasserstoffatom.

Die gestrichelten Linien in den Formeln in diesem Dokument stellen jeweils die Bindung zwischen einem Substituenten und dem zugehörigen Molekülrest dar.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.
Als "aliphatisch" wird ein Amin bezeichnet, dessen Aminogruppe an einen aliphatischen, cycloaliphatischen oder arylaliphatischen Rest gebunden ist; entsprechend wird diese Gruppe als aliphatische Aminogruppe bezeichnet.
Als "aromatisch" wird ein Amin bezeichnet, dessen Aminogruppe an einen aromatischen Rest gebunden ist; entsprechend wird diese Gruppe als aromatische Aminogruppe bezeichnet.
Als "Aminwasserstoff" werden die Wasserstoffatome von primären und sekundären Aminogruppen bezeichnet.
Als "nicht einbaubarer Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.
Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und in Ahnlehnung an DIN EN ISO 3219 beschrieben bestimmt wird.

Bevorzugt weist das Amin mit mindestens einer Aminogruppe der Formel (I) eine, zwei oder drei, besonders bevorzugt eine oder zwei, Aminogruppen der Formel (I) auf. Diese Amine sind besonders niedrigviskos.
Ein Amin mit nur einer Aminogruppe der Formel (I) weist bevorzugt zusätzlich mindestens eine weitere gegenüber Epoxidgruppen reaktive Gruppe auf, insbesondere eine weitere sekundäre Aminogruppe. Besonders bevorzugt trägt die weitere sekundäre Aminogruppe einen Benzylrest oder einen Hydroxybenzylrest. Damit lassen sich Eigenschaften des Amins wie Viskosität oder Reaktivität mit Epoxidgruppen beeinflussen.

Bevorzugt ist das Amin mit mindestens einer Aminogruppe der Formel (I) frei von primären Aminogruppen. Solche Amine sind besonders geeignet als Härter für Epoxidharze, da bei der Aushärtung keine Blushing-Effekte auftreten.

Bevorzugt steht die Dialkylaminogruppe der Aminogruppe der Formel (I) in para-Stellung.

Bevorzugt stehen Y für ein Wasserstoffatom, R¹ und R² für je eine Methylgruppe und die tertiäre Aminogruppe steht in para-Stellung. Eine solche Aminogruppe der Formel (I) stellt eine Aminogruppe der Formel (I a) dar. 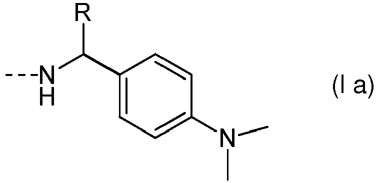 In der Formel (I a) weist R die bereits genannten Bedeutungen auf. Amine mit Aminogruppen der Formel (Ia) weisen besonders niedrige Viskositäten auf.

Das Amin mit mindestens einer Aminogruppe der Formel (I) stellt bevorzugt entweder ein Amin der Formel (II) dar, 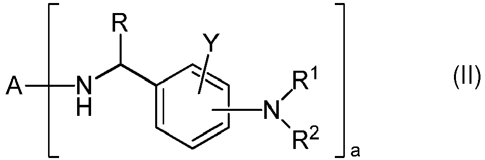 wobei
A für einen a-wertigen Kohlenwasserstoffrest mit einem Molekulargewicht im Bereich von 28 bis 5000 g/mol, welcher gegebenenfalls Ethergruppen, Amino-gruppen, Hydroxylgruppen oder Mercaptogruppen aufweist, steht;
a für eine ganze Zahl von 1 bis 3, bevorzugt für 1 oder 2, steht; und
R, R¹, R² und Y die bereits genannten Bedeutungen aufweisen;
oder es stellt ein Addukt eines Amins der Formel (II) mit mindestens einer Verbindung enthaltend mindestens eine, bevorzugt mindestens zwei, gleiche oder verschiedene Reaktivgruppen ausgewählt aus der Gruppe bestehend aus Epoxid-, Episulfid-, Aziridin-, Cyclocarbonat-, Isocyanat-, Isothiocyanat-, Acryl-, Methacryl- und Acrylamidgruppen, dar.

Bevorzugt steht A für einen a-wertigen Kohlenwasserstoffrest mit einem Molekulargewicht im Bereich von 28 bis 500 g/mol, welcher gegebenenfalls Ethergruppen oder primäre oder sekundäre Aminogruppen aufweist.

Besonders bevorzugt steht A entweder für
- einen a-wertigen Alkyl-, Cycloalkyl- oder Arylalkylrest mit 2 bis 20, insbesondere 2 bis 12, C-Atomen; oder für
- einen a-wertigen Polyalkylenaminrest mit 1 bis 10, insbesondere 1 bis 7, sekundären Aminogruppen, wobei als Alkylen insbesondere Ethylen, n-Propylen oder Hexamethylen vorhanden sind; oder für
- einen a-wertigen Polyoxyalkylenrest mit 1 bis 7 Ethergruppen, wobei als Alkylen insbesondere Ethylen oder Isopropylen vorhanden sind;.
wobei diese Reste eine oder zwei, bevorzugt eine, primäre oder sekundäre aliphatische Aminogruppen aufweisen können.

Insbesondere steht A für den a-wertigen Kohlenwasserstoffrest eines Amins nach Entfernung von a primären aliphatischen Aminogruppen, wobei das Amin ausgewählt ist aus der Gruppe bestehend aus 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,12-Dodecandiamin, 1,4-Diaminocyclohexan, Bis-(4-aminocyclohexyl)-methan, Bis-(4-amino-3-methylcyclohexyl)methan, 1-Amino-3-aminomethyl-3,5,5-trimethylcyclohexan (= Isophorondiamin oder IPDA), 1,3-Bis-(aminomethyl)cyclohexan, 2,5(2,6)-Bis-(aminomethyl)bicyclo[2.2.1]heptan (NBDA), 3(4),8(9)-Bis-(aminomethyl)-tricyclo[5.2.1.0^{2,6}]decan, 1,3-Bis-(aminomethyl)-benzol, Bis-hexamethylentriamin (BHMT), Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis(3-aminopropyl)ethylendiamin (N4-Amin) und Polyoxyalkylen-Diamine und Polyoxyalkylen-Triamine mit einem Molekulargewicht von 200 bis 500 g/mol.
Amine der Formel (II) mit den bevorzugten Kohlenwasserstoffresten A sind besonders gut verträglich mit Epoxidharzen und härten ohne Blushing-Effekte schnell zu qualitativ hochwertigen Filmen aus.

In einer ganz besonders bevorzugten Ausführungsform steht A für den Kohlenwasserstoffrest von MPMD oder 1,6-Hexandiamin oder TMD oder 1,3-Bis-(aminomethyl)benzol oder 1,3-Bis-(aminomethyl)cyclohexan oder Bis-(4-aminocyclohexyl)-methan oder Isophorondiamin nach Entfernung der primären Aminogruppen. Amine der Formel (II) mit diesen Resten A ergeben bei der Aushärtung mit Epoxidharzen hohe Härten und Beständigkeiten.
In einer weiteren ganz besonders bevorzugten Ausführungsform steht A für den Kohlenwasserstoffrest von DETA, TETA, TEPA, PEHA, HEPA, DPTA, N3-Amin oder N4-Amin nach Entfernung der primären Aminogruppen. Amine der Formel (II) mit diesen Resten A ergeben bei der Aushärtung mit Epoxidharzen besonders hohe Vernetzungsdichten und besonders hohe Härten und Beständigkeiten.
In einer weiteren ganz besonders bevorzugten Ausführungsform steht A für den Kohlenwasserstoffrest eines Polyoxyalkylen-Diamins oder Polyoxyalkylen-Triamins mit einem Molekulargewicht von 200 bis 500 g/mol nach Entfernung der primären Aminogruppen. Amine der Formel (II) mit diesen Resten A ergeben bei der Aushärtung mit Epoxidharzen besonders hohe Schlagzähigkeiten.

In einer Ausführungsform der Erfindung stellt das Amin mit mindestens einer Aminogruppe der Formel (I) ein Addukt eines Amins der Formel (II) mit mindestens einer Verbindung mit den vorgängig erwähnten Reaktivgruppen dar. Dazu werden die Aminogruppen gegenüber den vorgängig erwähnten Reaktivgruppen im stöchiometrischen Überschuss eingesetzt, insbesondere im Bereich von 2 bis 10 Aminogruppen pro vorgängig erwähnte Reaktivgruppe. Auf diese Weise sind Addukte mit mindestens einer Aminogruppe der Formel (I) erhältlich. Weist das zur Adduktierung eingesetzte Amin der Formel (II) primäre Aminogruppen auf, so reagieren diese bevorzugt mit den vorgängig erwähnten Reaktivgruppen.
Solche Addukte werden unter bekannten Bedingungen, wie sie für Reaktionen zwischen den beteiligten Reaktivgruppen typisch sind, hergestellt. Die Herstellung erfolgt unter Verwendung eines Lösemittels oder bevorzugt lösemittelfrei. Gegebenenfalls können Hilfsstoffe wie beispielsweise Katalysatoren, Initiatoren oder Stabilisatoren mitverwendet werden.

Insbesondere geeignete Verbindungen mit den genannten Reaktivgruppen sind
- Mono- oder Polyepoxide, insbesondere Epoxidharze oder Reaktivverdünner für Epoxidharze, wie sie im folgenden als Bestandteil einer Epoxidharz-Zusammensetzung beschrieben sind, sowie weiterhin Methylglycidylether, Ethylglycidylether und Propylglycidylether;
- monomere und oligomere Polyisocyanate, sowie mehr als eine Isocyanatgruppe aufweisende Umsetzungsprodukte von Polyisocyanaten mit Polyolen;
- mehr als eine Acryl-, Methacryl- oder Acrylamidgruppe tragende Verbindungen wie Tris-(2-hydroxyethyl)-isocyanurat-tri(meth)acrylat, Tris-(2-hydroxyethyl)-cyanurat-tri(meth)acrylat, N,N',N"-Tris-(meth)acryloyl-perhydrotriazin; Acrylate und Methacrylate von aliphatischen Polyethern, Polyestern, Novolaken, Phenolen, aliphatischen oder cycloaliphatischen Alkoholen, Glykolen und Polyesterglykolen sowie mono- und polyalkoxylierten Derivaten der vorgenannten Verbindungen, Addukte aus Polyepoxiden mit Acryl- und Methacrylsäure, Polyurethan(meth)acrylate und Acrylamide wie N,N'-Methylen-bis-acrylamid;
- sowie heterofunktionelle, das heisst mindestens zwei verschiedene der vorgenannten Reaktivgruppen tragende, Verbindungen.

In einer bevorzugten Ausführungsform ist das Addukt ein Addukt eines Amins der Formel (II) mit mindestens einem Mono- oder Polyepoxid, insbesondere mit mindestens einem Mono- oder Diepoxid.

Bevorzugt stellt das Amin mit mindestens einer Aminogruppe der Formel (I) ein Amin der Formel (II) dar. Ein Amin der Formel (II) weist eine besonders niedrige Viskosität auf.

Bevorzugt weist das Amin der Formel (II) eine Viskosität, gemessen bei 20 °C, im Bereich von 150 bis 2000 mPa·s, besonders bevorzugt im Bereich von 150 bis 1500 mPa·s, und insbesondere im Bereich von 150 bis 1000 mPa·s, auf.

Weiterhin betrifft die vorliegende Erfindung ein Verfahren zur Herstellung eines Härters wie er vorhergehend beschrieben worden ist, wobei das Amin mit mindestens einer Aminogruppe der Formel (I) durch die reduktive Alkylierung mindestens eines primären Amins mit mindestens einer Carbonylverbindung der Formel (III) erhalten wird. 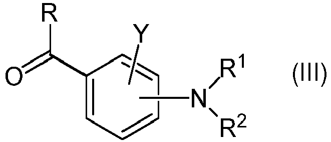 In der Formel (III) weisen R, R¹, R² und Y die bereits genannten Bedeutungen auf.

Als Carbonylverbindung der Formel (III) geeignet sind Aldehyde, insbesondere 4-Dimethylaminobenzaldehyd, 4-Dimethylamino-2-methyl-benzaldehyd, 4-Di-methylamino-2-methoxy-benzaldehyd, 4-Diethylaminobenzaldehyd, 4-Dibutylaminobenzaldehyd, 4-(N-Pyrrolidino)-benzaldehyd, 4-(N-Piperidino)-benzaldehyd, 3-Dimethylaminobenzaldehyd, 2-Dimethylaminobenzaldehyd, sowie weiterhin Ketone, insbesondere 4'-Dimethylaminoacetophenon, 4'-Diethylaminoacetophenon, 4'-(N-Pyrrolidino)-acetophenon, 4'-(N-Piperidino)-acetophenon und 4-Dimethylaminobenzophenon.
Bevorzugt sind 4-Dimethylaminobenzaldehyd, 4-Diethylaminobenzaldehyd, 4-Dibutylaminobenzaldehyd, 4'-Dimethylaminoacetophenon und 4-Dimethylaminobenzophenon.
Am meisten bevorzugt ist 4-Dimethylaminobenzaldehyd, welches technisch besonders gut verfügbar ist und eine geringe Toxizität aufweist.

Ein primäres Amin kann auch mit einer Mischung aus mindestens einer Carbonylverbindung der Formel (III) und mindestens einer weiteren Carbonylverbindung reduktiv alkyliert werden, insbesondere mit einer Mischung enthaltend Benzaldehyd und/oder Salicylaldehyd.

Die Carbonylverbindung wird in Bezug auf die primären Aminogruppen bevorzugt stöchiometrisch eingesetzt, wobei Amine mit mindestens einer Aminogruppe der Formel (I) erhalten werden, die frei sind von primären Aminogruppen.
Die reduktive Alkylierung wird geeigneterweise in Anwesenheit von Wasserstoff und unter erhöhtem Druck durchgeführt. Sie kann direkt mit molekularem Wasserstoff oder indirekt durch Wasserstoff-Transfer von anderen Reagentien erfolgen. Bevorzugt wird molekularer Wasserstoff verwendet. Dabei werden die Bedingungen vorteilhaft so gewählt, dass einerseits die umzusetzenden primären Aminogruppen möglichst vollständig reduktiv alkyliert werden und andererseits möglichst keine anderen Bestandteile des Amins und der Carbonylverbindung hydriert oder zersetzt werden. Bevorzugt wird bei einem WasserstoffDruck von 5 bis 100 bar, einer Temperatur von 40 bis 120 °C und in Anwesenheit eines geeigneten Katalysators gearbeitet. Als Katalysatoren bevorzugt sind Palladium auf Kohle (Pd/C), Platin auf Kohle (Pt/C), Adams-Katalysator und Raney-Nickel, insbesondere Palladium auf Kohle und Platin auf Kohle.

Ein Amin der Formel (II) kann besonders vorteilhaft durch die reduktive Alkylierung eines Amins der Formel (IV) mit mindestens einer Carbonylverbindung der Formel (III) erhalten werden.

A⁅NH₂]ₐ (IV)

In der Formel (IV) weisen A und a die bereits genannten Bedeutungen auf.

Als Amin der Formel (IV) geeignet sind in einer ersten Ausführungsform primäre aliphatische Polyamine, die als Härter für Epoxidharze bekannt sind, insbesondere die Folgenden:
- aliphatische, cycloaliphatische oder arylaliphatische primäre Diamine, wie insbesondere Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis-(4-aminocyclohexyl)-methan, Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5, 5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)-bicyclo[2.2.1]heptan (NBDA), 3(4), 8(9)-Bis-(aminomethyl)-tricyclo[5.2.1.0^{2,6}]decan, 1,4-Diamino-2,2,6-trimethylcyclohexan (TMCDA), 1,8-Menthandiamin, 3,9-Bis-(3-aminopropyl)-2,4,8,10-tetra-oxaspiro[5.5]undecan, 1,3- Bis-(aminomethyl)benzol und 1,4-Bis-(aminomethyl)benzol;
- aliphatische, cycloaliphatische oder arylaliphatische primäre Triamine wie 4-Aminomethyl-1,8-octandiamin, 1,3,5-Tris-(aminomethyl)benzol und 1,3,5-Tris-(aminomethyl)cyclohexan;
- Ethergruppen-haltige aliphatische primäre Diamine, wie insbesondere Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin und höhere Oligomere dieser Diamine, Bis-(3-aminopropyl)polytetrahydrofurane und andere Polytetrahydrofuran-diamine, sowie Polyoxyalkylen-Diamine. Letztere stellen typischerweise Produkte aus der Aminierung von Polyoxyalkylen-Diolen dar und sind beispielsweise erhältlich unter dem Namen Jeffamine® (von Huntsman), unter dem Namen Polyetheramine (von BASF) oder unter dem Namen PC Amine® (von Nitroil). Insbesondere geeignete Polyoxyalkylen-Diamine sind Jeffamine® D-230, Jeffamine® D-400, Jeffamine® D-2000, Jeffamine® D-4000, Jeffamine® XTJ-511, Jeffamine® ED-600, Jeffamine® ED-900, Jeffamine® ED-2003, Jeffamine® XTJ-568, Jeffamine® XTJ-569, Jeffamine® XTJ-523, Jeffamine® XTJ-536, Jeffamine® XTJ-542, Jeffamine® XTJ-559, Jeffamine® EDR-104, Jeffamine® EDR-148, Jeffamine® EDR-176; Polyetheramine D 230, Polyetheramine D 400 und Polyetheramine D 2000, PC Amine® DA 250, PC Amine® DA 400, PC Amine® DA 650 und PC Amine® DA 2000;
- primäre Polyoxyalkylen-Triamine, welche typischerweise Produkte aus der Aminierung von Polyoxyalkylen-Triolen darstellen und beispielsweise erhältlich sind unter dem Namen Jeffamine® (von Huntsman), unter dem Namen Polyetheramine (von BASF) oder unter dem Namen PC Amine® (von Nitroil), wie insbesondere Jeffamine® T-403, Jeffamine® T-3000, Jeffamine® T-5000, Polyetheramine T 403, Polyetheramine T 5000 und PC Amine® TA 403;
- tertiäre Aminogruppen aufweisende Polyamine mit zwei primären aliphatischen Aminogruppen, wie insbesondere N,N'-Bis-(aminopropyl)piperazin, N,N-Bis-(3-aminopropyl)methylamin, N,N-Bis-(3-aminopropyl)ethylamin, N,N-Bis-(3-aminopropyl)propylamin, N,N-Bis-(3-aminopropyl)cyclohexylamin, N,N-Bis-(3-aminopropyl)-2-ethyl-hexylamin, sowie die Produkte aus der doppelten Cyanoethylierung und nachfolgender Reduktion von Fettaminen, welche abgeleitet sind von natürlichen Fettsäuren, wie N,N-Bis-(3-aminopropyl)-dodecylamin und N,N-Bis-(3-aminopropyl)-talgalkylamin, erhältlich als Triameen® Y12D und Triameen® YT (von Akzo Nobel);
- tertiäre Aminogruppen aufweisende Polyamine mit drei primären aliphatischen Aminogruppen, wie insbesondere Tris-(2-aminoethyl)amin, Tris-(2-aminopropyl)amin und Tris-(3-aminopropyl)amin;
- sekundäre Aminogruppen aufweisende Polyamine mit zwei primären aliphatischen Aminogruppen, wie insbesondere 3-(2-Aminoethyl)aminopropylamin, Bis-hexamethylentriamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (sogenanntes "higher ethylenepolyamine", HEPA), Produkte aus der Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis-(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin und N,N'-Bis-(3-amino-1-ethylpropyl)-2-methyl-1,5-pentandiamin.

Als Amin der Formel (IV) geeignet sind in einer weiteren Ausführungsform Amine mit nur einer primären aliphatischen Aminogruppe, insbesondere die Folgenden:
- Monoamine, wie insbesondere Benzylamin, Cyclohexylamin, 2-Phenylethylamin, 2-Methoxyphenylethylamin, 4-Methoxyphenylethylamin, 3,4-Dimethoxyphenylethylamin (Homoveratrylamin), Methylamin, Ethylamin, Propylamin, Isopropylamin, 1- und 2-Butylamin, Isobutylamin, tert.-Butylamin, 3-Methyl-2-butylamin, 1-Hexylamin, 1-Octylamin, 2-Ethyl-1-hexylamin, 2-Methoxy-1-ethylamin, 2-Ethoxy-1-ethylamin, 3-Methoxy-1-propylamin, 3-Ethoxy-1-propylamin, 3-(2-Ethylhexyloxy)propylamin, 3-(2-Methoxyethoxy)propylamin;
- primäre und sekundäre Aminogruppen aufweisende Polyamine, wie insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, N-(2-Aminoethyl)piperazin, N-Methyl-1,3-propandiamin, N-Butyl-1,3-propandiamin, N-(2-Ethylhexyl)-1,3-propandiamin, N-Cyclohexyl-1,3-propandiamin, 3-Methylamino-1-pentylamin, 3-Ethylamino-1-pentylamin, 3-Cyclohexylamino-1-pentylamin, Fettdiamine wie N-Cocoalkyl-1,3-propandiamin und Produkte aus der Michael-artigen Additionsreaktion von primären aliphatischen Diaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1;
- primäre und tertiäre Aminogruppen aufweisende Polyamine, wie insbesondere 3-(Dimethylamino)-1-propylamin;
- Aminoalkohole, wie insbesondere 3-Amino-1-propanol, 2-Amino-1-butanol, 6-Amino-1-hexanol, Aminopropyldiethanolamin (APDEA), 4-(2-Aminoethyl)-2-hydroxyethylbenzol, 3-Aminomethyl-3,5,5-trimethyl-cyclohexanol, 2-(2-Aminoethoxy)ethanol, 2-(2-(2-Aminoethoxy)ethoxy)ethanol, 3-(2-Hydroxy-ethoxy)propylamin und 3-(2-(2-Hydroxyethoxy)-ethoxy)propylamin;
- Aminomercaptane, wie insbesondere 2-Aminoethanthiol (Cysteamin), 3-Aminopropanthiol, 4-Amino-1-butanthiol und 6-Amino-1-hexanthiol.

Als Amin der Formel (IV) bevorzugt sind Amine mit einem Molekulargewicht bis 500 g/mol, welche gegebenenfalls Ethergruppen aufweisen.

Besonders bevorzugt ist das Amin der Formel (IV) ausgewählt aus der Gruppe bestehend aus MPMD, C11-Neodiamin, 1,6-Hexandiamin, 2,5-Dimethyl-1,6-hexandiamin, TMD, 1,12-Dodecandiamin, 1,4-Diaminocyclohexan, Bis-(4-aminocyclohexyl)methan, Bis-(4-amino-3-methylcyclohexyl)methan, Isophorondiamin, 1,3-Bis-(aminomethyl)cyclohexan, NBDA, 3(4),8(9)-Bis-(aminomethyl)-tricyclo[5.2.1.0^{2,6}]decan, 1,3-Bis-(aminomethyl)benzol, BHMT, DETA, TETA, TEPA, PEHA, HEPA, DPTA, N3-Amin, N4-Amin, Polyoxyalkylen-Diaminen und Polyoxyalkylen-Triaminen mit einem Molekulargewicht von 200 bis 500 g/mol, insbesondere den kommerziellen Typen Jeffamine® D-230, Jeffamine® D-400 und Jeffamine® T-403 (von Huntsman).

Die Herstellung von Aminen mit mindestens einer Aminogruppe der Formel (I) durch reduktive Alkylierung auf die beschriebene Weise ist für die Verwendung als Härter für Epoxidharze besonders vorteilhaft, da sehr selektiv primäre Aminogruppen alkyliert werden, während sekundäre Aminogruppen kaum weiter alkyliert werden. Die Produkte aus der beschriebenen Herstellung können deshalb nach der reduktiven Alkylierung ohne weitere Aufbereitung zur Aushärtung von Epoxidharzen in der beschriebenen Weise verwendet werden.

Amine mit mindestens einer Aminogruppe der Formel (I) lassen sich auch auf andere Weise als durch reduktive Alkylierung erhalten, insbesondere durch Umsetzung von primären Aminen mit entsprechenden Chloriden oder Bromiden in einem geeigneten Verhältnis. Dabei entstehen aber Reaktionsgemische, welche typischerweise einen erheblichen Anteil an doppelt alkylierten Amino-gruppen aufweisen.

Ein weiterer Gegenstand der Erfindung ist die Verwendung des beschriebenen Härters zum Aushärten von mindestens einem Epoxidharz. Dazu wird der Härter mit dem Epoxidharz auf eine geeignete Weise vermischt.

Der beschriebene Härter weist besonders vorteilhafte Eigenschaften auf. Er ist wenig flüchtig und geruchsarm und weist eine so geringe Reaktivität gegenüber CO₂ auf, dass er - im Gegensatz zu vielen aus dem Stand der Technik bekannten Härtern - an der Luft weder zur Bildung von Krusten noch zu Ausfällungen oder Viskositätserhöhungen neigt. Der Härter ist mit den üblichen kommerziellen Epoxidharzen gut verträglich und verarbeitbar und härtet bei Umgebungstemperatur überraschend schnell und ohne störende Blushing-Effekte zu ausgehärteten Zusammensetzungen von hoher Härte und Beständigkeit und überraschend geringer Verfärbungsneigung aus. Die geringe Verfärbungsneigung ist insbesondere für Anwendungen, bei welchen das Produkt dauerhaft sichtbar bleibt, beispielsweise als Bodenbelag, eine wichtige Voraussetzung. Besonders vorteilhaft ist auch die niedrige Viskosität der Härter. Sie sind auch ohne nicht-einbaubare Verdünner in selbstverlaufenden Beschichtungen verwendbar und dadurch insbesondere auch für emissionsarme Systeme geeignet.

Der beschriebene Härter kann neben dem Amin mit mindestens einer Amino-gruppe der Formel (I) weitere, zur Aushärtung von Epoxidharzen geeignete Verbindungen enthalten, insbesondere die Folgenden:
- die vorgängig beschriebenen Amine der Formel (IV);
- sekundäre aliphatische Polyamine, wie insbesondere die Produkte aus der reduktiven Alkylierung der vorgängig beschriebenen Amine der Formel (IV) mit andern Carbonylverbindungen, insbesondere mit Benzaldehyd und/oder Salicylaldehyd und/oder 3-Nitrobenzaldehyd und/oder 3-Pyridincarbaldehyd; weiterhin N,N'-Dibutyl-ethylendiamin, N,N'-Di-tert.butyl-ethylendiamin, N,N'-Diethyl-1 ,6-hexandiamin, 1-(1-Methylethyl-amino)-3-(1-methylethyl-aminomethyl)-3,5,5-trimethylcyclohexan (Jefflink® 754 von Huntsman), N⁴-Cyclohexyl-2-methyl-N²-(2-methylpropyl)-2,4-pentandiamin, N,N'-Dialkyl-1,3-bis-(aminomethyl)benzol, insbesondere N,N'-Dibenzyl-1,3-bis-(aminomethyl)-benzol, Bis-(4-(N-3-butylamino)-cyclohexyl)methan (Clearlink® 1000 von UOP), dialkyliertes DETA oder TETA oder TEPA oder PEHA oder N3- oder N4-Amin, insbesondere die dibenzylierten, gegebenenfalls Phenolgruppen aufweisenden Typen, weiterhin styrolisierte Polyamine wie beispielsweise styrolisiertes 1,3-Bis-(aminomethyl)benzol, kommerziell erhältlich als Gask-amine® 240 (von Mitsubishi Gas Chemical), N-alkylierte Polyetheramine, beispielsweise die Jeffamine®-Typen SD-231, SD-401, ST-404 und SD-2001 (von Huntsman), sowie Produkte aus der Michael-artigen Additionsreaktion von primären aliphatischen Polyaminen mit Michaelakzeptoren wie Maleinsäurediestern, Fumarsäurediestern, Citraconsäurediestern, Acrylsäureestern, Methacrylsäureestern, Zimtsäureestern, Itaconsäurediestern, Vinylphosphonsäurediestern, Vinylsulfonsäurearylestern, Vinylsulfonen, Vinylnitrilen, Nitroalkylenen oder Knoevenagel-Kondensationsprodukten wie beispielsweise solchen aus Malonsäurediestern und Aldehyden wie Formaldehyd, Acetaldehyd oder Benzaldehyd;
- aromatische Polyamine, wie insbesondere m- und p-Phenylendiamin, 4,4'-, 2,4'- und 2,2'-Diaminodiphenylmethan, 3,3'-Dichloro-4,4'-diaminodiphenylmethan (MOCA), 2,4- und 2,6-Toluylendiamin, Mischungen von 3,5-Dimethylthio-2,4- und -2,6-toluylendiamin (erhältlich als Ethacure® 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-5,5'-methylendianthranilat, 1,3-Propylen-bis-(4-aminobenzoat), 1,4-Butylen-bis-(4-aminobenzoat), Polytetramethylenoxid-bis-(4-aminobenzoat) (erhältlich als Versalink® von Air Products), 1,2-Bis-(2-aminophenylthio)-ethan, 2-Methylpropyl-(4-chloro-3,5-diaminobenzoat) und tert.Butyl-(4-chloro-3,5-diaminobenzoat);
- Amin/Epoxid-Addukte, insbesondere Addukte aus den genannten Aminen mit Diepoxiden im Molverhältnis von mindestens 2/1, insbesondere im Molverhältnis von 2/1 bis 6/1, oder mit Monoepoxiden im Molverhältnis von mindestens 1/1, sowie Umsetzungsprodukte aus Aminen und Epichlorhydrin, insbesondere jenes von 1,3-Bis-(aminomethyl)benzol, kommerziell erhältlich als Gaskamine® 328 (von Mitsubishi Gas Chemical);
- Polyamidoamine, welche Umsetzungsprodukte aus einer ein- oder mehrwertigen Carbonsäure, beziehungsweise deren Ester oder Anhydride, insbesondere einer Dimerfettsäure, und einem im stöchiometrischen Überschuss eingesetzten aliphatischen, cycloaliphatischen oder aromatischen Polyamin, insbesondere einem Polyalkylenamin wie beispielsweise DETA oder TETA, darstellen, insbesondere die kommerziell erhältlichen Polyamidoamine Versamid® 100, 125, 140 und 150 (von Cognis), Aradur® 223, 250 und 848 (von Huntsman), Euretek®3607 und 530 (von Huntsman) und Beckopox® EH 651, EH 654, EH 655, EH 661 und EH 663 (von Cytec); und
- Mannich-Basen, teilweise auch Phenalkamine genannt, welche Umsetzungsprodukte einer Mannich-Reaktion von Phenolen, insbesondere Cardanol, Nonylphenol oder tert.Butylphenol, mit Aldehyden, insbesondere Formaldehyd, und Polyaminen darstellen, insbesondere die kommerziell erhältlichen Mannich-Basen Cardolite® NC-541, NC-557, NC-558, NC-566, Lite 2001 und Lite 2002 (von Cardolite), Aradur® 3440, 3441, 3442 und 3460 (von Huntsman), Accelerator 2950 (von Huntsman) und Beckopox® EH 614, EH 621, EH 624, EH 628 und EH 629 (von Cytec);
- flüssige Mercaptan-terminierte Polysulfid-Polymere, bekannt unter dem Markennamen Thiokol® (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 und LP-2; sowie weiterhin bekannt unter dem Markennamen Thioplast® (von Akzo Nobel), insbesondere die Typen G 10, G 112, G 131, G 1, G 12, G 21, G 22, G 44 und G 4;
- Mercaptan-terminierte Polyoxyalkylen-Ether, erhältlich beispielsweise durch Umsetzung von Polyoxyalkylendi- und -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure® (von Cognis), insbesondere die Typen WR-8, LOF und 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat), sowie die Veresterungsprodukte von Polyoxyalkylendiolen und -triolen, ethoxyliertem Trimethylolpropan und Polyester-Diolen mit Thiocarbonsäuren wie Thioglykolsäure und 2- oder 3-Mercaptopropionsäure;
- weitere Mercaptogruppen aufweisende Verbindungen, wie insbesondere 2,4,6-Trimercapto-1,3,5-triazin, 2,2'-(Ethylendioxy)diethanthiol (Triethylenglykol-dimercaptan) und Ethandithiol.
Davon bevorzugt sind DAMP, MPMD, C11-Neodiamin, 1,6-Hexandiamin, 2,5-Dimethyl-1,6-hexandiamin, TMD, 1,12-Dodecandiamin, 1,4-Diaminocyclohexan, Bis-(4-aminocyclohexyl)methan, Bis-(4-amino-3-methylcyclohexyl)methan, IPDA, 3(4),8(9)-Bis-(aminomethyl)-tricyclo[5.2.1.0^{2,6}]decan, 1,3-Bis-(aminomethyl)benzol, N,N'-Dibenzyl-1,3-bis-(aminomethyl)benzol, 1,3-Bis-(aminomethyl)cyclohexan, Gaskamine® 240, NBDA, dibenzyliertes DETA, dibenzyliertes TETA, dibenzyliertes N3-Amin und dibenzyliertes N4-Amin, wobei diese dibenzylierten Amine gegebenenfalls Phenolgruppen aufweisen, Polyoxyalkylen-Diamine und -Triamine mit einem Molekulargewicht im Bereich von 200 bis 500 g/mol, insbesondere die Typen Jeffamine® D-230, Jeffamine® D-400 und Jeffamine® T-403, weiterhin Amin/Epoxid-Addukte, insbesondere Gaskamine® 328, sowie Mannich-Basen.

Der erfindungsgemässe Härter kann weiterhin mindestens einen Beschleuniger enthalten. Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; weiterhin tertiäre Amine wie insbesondere 1,4-Diazabicyclo[2.2.2]octan, Benzyldimethylamin, α-Methylbenzyldimethylamin, Triethanolamin, Dimethylaminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diaza-bicyclo[5.4.0]undec-7-en, Guanidine wie insbesondere 1,1,3,3-Tetramethylguanidin, Phenole, insbesondere Bisphenole, Phenol-Harze und Mannich-Basen wie insbesondere 2-(Dimethylaminomethyl)phenol, 2,4,6-Tris-(dimethylaminomethyl)phenol und Polymere aus Phenol, Formaldehyd und N,N-Dimethyl-1,3-propandiamin, Phosphite wie insbesondere Di- und Triphenylphosphite, sowie Mercaptogruppen aufweisende Verbindungen, wie sie bereits vorgängig genannt wurden.
Bevorzugte Beschleuniger sind Salicylsäure und 2,4,6-Tris-(dimethylaminomethyl)phenol.

Der erfindungsgemässe Härter kann weiterhin mindestens einen nicht einbaubaren Verdünner enthalten, wie insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso®-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, Adipate, Sebacate, Phthalate, Benzoate, organische Phosphor- und Sulfonsäureester und Sulfonamide. Bevorzugt sind Benzylalkohol, Dodecylphenol, tert.Butylphenol, styrolisiertes Phenol und phenolgruppenhaltige aromatische Kohlenwasserstoffharze, insbesondere die Novares®-Typen LS 500, LX 200, LA 300 und LA 700 (von Rütgers).

Bevorzugt enthält der Härter keinen oder nur einen geringen Gehalt an nicht einbaubaren Verdünnern, besonders bevorzugt weniger als 25 Gewichts-%, insbesondere weniger als 15 Gewichts-% und am meisten bevorzugt weniger als 5 Gewichts-%. Insbesondere werden dem Härter keine nicht einbaubaren Verdünner zugesetzt.

Ein weiterer Gegenstand der Erfindung ist eine Epoxidharz-Zusammensetzung, enthaltend mindestens ein Epoxidharz und mindestens einen Härter wie vorgängig beschrieben.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.

Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf, im Gegensatz zu den sogenannten Festharzen, welche eine Glasübergangstemperatur oberhalb von 25°C aufweisen und sich zu bei 25°C schüttfähigen Pulvern zerkleinern lassen.
In einer Ausführungsform handelt es sich beim Flüssigharz um ein aromatisches Polyepoxid. Dafür geeignet sind beispielsweise Flüssigharze der Formel (V), 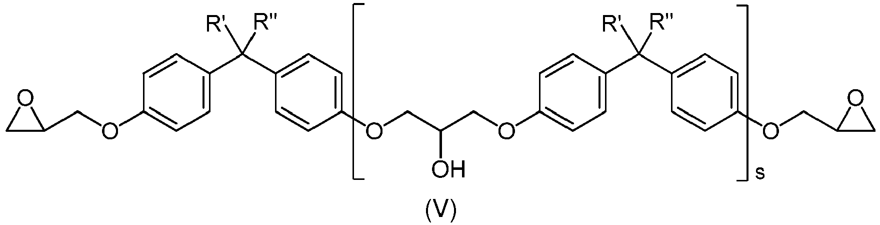 wobei R' und R" unabhängig voneinander jeweils für ein Wasserstoffatom oder für eine Methylgruppe stehen, und s im Mittel für einen Wert von 0 bis 1 steht. Bevorzugt sind solche Flüssigharze der Formel (V), bei denen der Index s im Mittel für einen Wert von kleiner als 0.2 steht.
Bei den Flüssigharzen der Formel (V) handelt es sich um Diglycidylether von Bisphenol-A, Bisphenol-F und Bisphenol-A/F, wobei A für Aceton und F für Formaldehyd steht, welche als Edukte zur Herstellung dieser Bisphenole dienen. Im Fall von Bisphenol-F können auch Stellungsisomere vorhanden sein, insbesondere abgeleitet von 2,4'- und 2,2'-Hydroxyphenylmethan.
Weitere geeignete aromatische Flüssigharze sind die Glycidylisierungsprodukte von
- Dihydroxybenzol-Derivaten wie Resorcin, Hydrochinon und Brenzkatechin;
- weiteren Bisphenolen oder Polyphenolen wie Bis-(4-hydroxy-3-methylphenyl)-methan, 2,2-Bis-(4-hydroxy-3-methylyphenyl)-propan (Bisphenol-C), Bis-(3,5-dimethyl-4-hydroxyphenyl)-methan, 2,2-Bis-(3,5-dimethyl-4-hydroxyphenyl)-propan, 2,2-Bis-(3,5-dibromo-4-hydroxyphenyl)-propan, 2,2-Bis-(4-hydroxy-3-tert.-butylphenyl)-propan, 2,2-Bis-(4-hydroxyphenyl)-butan (Bisphenol-B), 3,3-Bis-(4-hydroxyphenyl)-pentan, 3,4-Bis-(4-hydroxyphenyl)-hexan, 4,4-Bis-(4-hydroxyphenyl)-heptan, 2,4-Bis-(4-hydroxyphenyl)-2-methylbutan, 2,4-Bis-(3,5-dimethyl-4-hydroxyphenyl)-2-methylbutan, 1,1-Bis-(4-hydroxyphenyl)-cyclohexan (Bisphenol-Z), 1,1-Bis-(4-hydroxyphenyl)-3,3,5-trimethylcyclohexan (Bisphenol-TMC), 1,1-Bis-(4-hydroxyphenyl)-1-phenyl-ethan, 1,4-Bis[2-(4-hydroxyphenyl)-2-propyl]benzol) (Bisphenol-P), 1,3-Bis-[2-(4-hydroxyphenyl)-2-propyl]benzol) (Bisphenol-M), 4,4'-Dihydroxydiphenyl (DOD), 4,4'-Dihydroxybenzophenon, Bis-(2-hydroxynaphth-1-yl)-methan, Bis-(4-hydroxynaphth-1-yl)-methan 1,5-Dihydroxy-naphthalin, Tris-(4-hydroxyphenyl)methan, 1,1,2,2-Tetrakis-(4-hydroxyphenyl)ethan, Bis-(4-hydroxyphenyl)ether, Bis-(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis-(1-methylethyliden)]bisanilin (Bisanilin-P), 4,4'-[1,3-Phenylen-bis-(1-me-thylethyliden)]-bisanilin (Bisanilin-M).

Als Epoxidharz eignet sich auch ein aliphatisches oder cycloaliphatisches Polyepoxid, wie beispielsweise
- ein Glycidylether eines gesättigten oder ungesättigten, verzweigten oder unverzweigten, zyklischen oder offenkettigen C₂- bis C₃₀-Diols, wie beispielsweise Ethylenglykol, Propylenglykol, Butylenglykol, Hexandiol, Octandiol, ein Polypropylenglykol, Dimethylolcyclohexan, Neopentylglykol oder Dibromo-neopentylglykol;
- ein Glycidylether eines tri- oder tetrafunktionellen, gesättigten oder ungesättigten, verzweigten oder unverzweigten, zyklischen oder offenkettigen Polyols wie Rizinusöl, Trimethylolpropan, Trimethylolethan, Pentaerythrol, Sorbit oder Glycerin, sowie alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat und Triglycidylisocyanurat, sowie Umsetzungsprodukte von Epichlorhydrin und Hydantoin.
Als Epoxidharz möglich sind auch ein Bisphenol-A-, -F- oder -A/F-Festharz, welches ähnlich aufgebaut ist wie die bereits genannten Flüssigharze der Formel (V), aber anstelle des Index s einen Wert von 2 bis 12 aufweist, und eine Glasübergangstemperatur oberhalb von 25 °C aufweist.
Als Epoxidharz eignen sich schliesslich auch Epoxidharze aus der Oxidation von Olefinen, beispielsweise aus der Oxidation von Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz bevorzugt sind Flüssigharze auf der Basis eines Bisphenols, insbesondere auf der Basis von Bisphenol-A, Bisphenol-F- oder Bisphenol-A/F, wie sie kommerziell beispielsweise von Dow, Huntsman und Hexion erhältlich sind. Diese Flüssigharze weisen eine für Epoxidharze niedrige Viskosität und im ausgehärteten Zustand gute Eigenschaften als Beschichtung auf. Sie können gegebenenfalls in Kombination mit Bisphenol A-Festharz oder Bisphenol-F-Novolak-Epoxidharz vorhanden sein.

Das Epoxidharz kann einen Reaktivverdünner, insbesondere einen mindestens eine Epoxidgruppe aufweisenden Reaktivverdünner, enthalten. Als Reaktivverdünner geeignet sind beispielsweise die Glycidylether von ein- oder mehrwertigen Phenolen und aliphatischen oder cycloaliphatischen Alkoholen, wie insbesondere die bereits genannten Polyglycidylether von Di- oder Polyolen, sowie weiterhin insbesondere Phenylglycidylether, Kresylglycidylether, Benzylglycidylether, p-n-Butyl-phenylglycidylether, p-tert.Butyl-phenylglycidylether, Nonylphenylglycidylether, Allylglycidylether, Butylglycidylether, Hexylglycidylether, 2-Ethylhexylglycidylether, sowie Glycidylether von natürlichen Alkoholen, wie zum Beispiel C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, sowie - im ausgehärteten Zustand der Epoxidharz-Zusammensetzung - eine Reduktion der Glasübergangstemperatur und der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten nicht einbaubaren Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate und Reaktivgruppen-aufweisende Silikone;
- Polymere, wie beispielsweise Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere chlorsulfonierte Polyethylene und Fluor-haltige Polymere, Sulfonamid-modifizierte Melamine und gereinigte Montan-Wachse;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und Eisenoxide;
- die vorgenannten Beschleuniger;
- Rheologie-Modifizierer, wie insbesondere Verdickungsmittel, zum Beispiel Schichtsilikate wie Bentonite, Derivate von Rizinusöl, hydriertes Rizinusöl, Polyamide, Polyurethane, Harnstoffverbindungen, pyrogene Kieselsäuren, Celluloseether und hydrophob modifizierte Polyoxyethylene;
- Haftverbesserer, beispielsweise Organoalkoxysilane wie Aminosilane, Mercaptosilane, Epoxysilane, Vinylsilane, (Meth)acrylsilane, Isocyanatosilane, Carbamatosilane, Alkylsilane, S-(Alkylcarbonyl)mercaptosilane und Aldiminosilane, sowie oligomere Formen dieser Silane, insbesondere 3-Glycidoxypropyltrimethoxysilan, 3-Aminopropyltrimethoxysilan, N-(2-Aminoethyl)-3-aminopropyl-trimethoxysilan, N-(2-Aminoethyl)-N'-[3-(trimethoxysilyl)propyl]ethylendiamin, 3-Mercaptopropyltrimethoxysilan, 3-Isocyanatopropyltrimethoxysilan, 3-Ureidopropyltrimethoxysilan, 3-Chloropropyltrimethoxysilan, Vinyltrimethoxysilan, oder die entsprechenden Organosilane mit Ethoxygruppen anstelle der Methoxygruppen;
- Stabilisatoren gegen Oxidation, Wärme, Licht und UV-Strahlung;
- flammhemmende Substanzen, insbesondere Verbindungen wie Aluminiumhydroxid (Al(OH)₃; auch ATH für "Aluminiumtrihydrat" genannt), Magnesiumhydroxid (Mg(OH)₂; auch MDH für "Magnesiumdihydrat" genannt), Ammoniumsulfat ((NH₄)₂SO₄), Borsäure (B(OH)₃), Zinkborat, Zinkphosphat, Melaminborat und Melamincyanurat; Phosphor-haltige Verbindungen wie Ammoniumphosphat ((NH₄)₃PO₄), Ammoniumpolyphosphat, Melaminphosphat, Melaminpyrophosphat, Triphenylphosphat, Diphenylkresylphosphat, Trikresylphosphat, Triethylphosphat, Tris-(2-ethylhexyl)phosphat, Trioctylphosphat, Mono-, Bis- und Tris-(isopropylphenyl)phosphat, Resorcinol-bis(diphenylphosphat), Resorcinol-diphosphat-Oligomer, Tetraphenyl-resorcinol-diphosphit, Ethylendiamin-diphosphat und Bisphenol-A-bis(diphenylphosphat); Halogen-haltige Verbindungen wie Chloroalkylphosphate, insbesondere Tris-(chloroethyl)phosphat, Tris-(chloropropyl)phosphat und Tris-(dichloro-isopropyl)phosphat, polybromierte Diphenylether, insbesondere Decabromdiphenylether, polybromiertes Diphenyloxid, Tris-[3-Bromo-2,2-bis(bromo-methyl)propyl]phosphat, Tetrabromo-Bisphenol-A, Bis-(2,3-dibromopropyl-ether) von Bisphenol A, bromierte Epoxidharze, Ethylen-bis(tetrabromo-phtalimid), Ethylen-bis(dibromonorbornandicarboximid), 1,2-Bis-(tribromo-phenoxy)ethan, Tris-(2,3-dibromopropyl)isocyanurat, Tribromophenol, Hexabromocyclododecan, Bis-(hexachlorocyclopentadieno)cyclooctan und Chlorparaffine; sowie Kombinationen aus einer Halogen-haltigen Verbindung und Antimontrioxid (Sb₂O₃) oder Antimonpentoxid (Sb₂O₅);
- oberflächenaktive Substanzen, insbesondere Netzmittel, Verlaufsmittel, Entlüftungsmittel und Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und Katalysatoren, insbesondere Salicylsäure oder 2,4,6-Tris-(dimethylaminomethyl)phenol.
Bevorzugt enthält die Epoxidharz-Zusammensetzung keinen oder nur einen geringen Gehalt an nicht einbaubaren Verdünnern, besonders bevorzugt weniger als 10 Gewichts-%, insbesondere weniger als 5 Gewichts-%, am meisten bevorzugt weniger als 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen im Bereich von 0.5 bis 1.5, bevorzugt 0.7 bis 1.2.
Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Insbesondere ist die Epoxidharz-Zusammensetzung eine zweikomponentige Zusammensetzung, bestehend aus einer Harz-Komponente und einer Härter-Komponente, wobei das Epoxidharz Bestandteil der Harz-Komponente und der beschriebene Härter Bestandteil der Härter-Komponente ist.

Die Komponenten der zweikomponentigen Zusammensetzung werden jeweils in einem eigenen Gebinde gelagert. Weitere Bestandteile der zweikomponentigen Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der zweikomponentigen Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.
Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.
Mit der Vermischung der beiden Komponenten beginnt die Aushärtung durch chemische Reaktion, wie vorgängig beschrieben. Die Aushärtung erfolgt insbesondere bei Umgebungstemperatur. Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.

Ein weiterer Gegenstand der Erfindung ist somit auch eine ausgehärtete Zusammensetzung erhalten aus der Aushärtung einer Epoxidharz-Zusammensetzung wie im vorliegenden Dokument beschrieben.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips und Natursteine wie Granit oder Marmor;
- Metalle und Legierungen, wie Aluminium, Eisen, Stahl und Buntmetalle, sowie oberflächenveredelte Metalle und Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Polyester, Epoxidharze, Polyurethane (PUR), Polyoxymethylen (POM), Polyolefine (PO), Polyethylen (PE) oder Polypropylen (PP), Ethylen/Propylen-Copolymere (EPM) und Ethylen/Propylen/Dien-Terpolymere (EPDM), wobei die Kunststoffe bevorzugt mittels Plasma, Corona oder Flammen oberflächenbehandelt sein können;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- beschichtete Substrate, wie pulverbeschichtete Metalle oder Legierungen;
- Farben und Lacke, insbesondere Automobildecklacke.

Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten oder dergleichen, wobei dabei entstehende Stäube vorteilhaft abgesaugt werden, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundwerkstoff (Composite), Vergussmasse, Dichtstoff, Klebstoff, Belag, Beschichtung, Anstrich, Lack, Versiegelung, Grundierung oder Primer. Insbesondere verwendbar ist sie als Vergussmasse, Dichtstoff und Klebstoff, wie beispielsweise als Elektrovergussmasse, Abdichtungsmasse, Karrosserieklebstoff, Sandwichelementklebstoff, Halbschalenklebstoff, beispielsweise für Rotorblätter von Windkraftanlagen, Brückenelementklebstoff oder Verankerungsklebstoff; sowie weiterhin als Belag, Beschichtung, Anstrich, Lack, Versiegelung, Grundierung und Primer für Bau- und Industrieanwendungen, wie insbesondere als Bodenbelag und Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden, wobei diese Beschichtungen die jeweiligen Substrate insbesondere gegen Korrosion, Abrasion, Feuchtigkeit, Wasser- und/oder Salzeinwirkung oder Chemikalien schützen; sowie weiterhin als Voranstrich, Haftanstrich, Korrosionsschutz-Primer oder zur Hydrophobierung von Oberflächen. Insbesondere geeignet ist die beschriebene Zusammensetzung auch als Beschichtung für sogenannt schweren Korrosionsschutz im und am Wasser, insbesondere auch im und am Meerwasser. Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft verwendbar ist die beschriebene Epoxidharz-Zusammensetzung als Beschichtung. Als Beschichtung werden dabei flächig aufgebrachte Beläge aller Art verstanden, insbesondere auch Anstriche, Lacke, Versiegelungen, Grundierungen und Primer, wie vorgängig beschrieben. Insbesondere vorteilhaft verwendbar ist die beschriebene Epoxidharz-Zusammensetzung in emissionsarmen Systemen mit Öko-Gütesiegeln, beispielsweise nach Emicode (EC1 Plus), AgBB, DIBt, Der Blaue Engel, AFSSET, RTS (M1) und US Green Building Council (LEED), sowie für Beschichtungen, welche gegenüber mechanischer Belastung, Feuchtigkeit, Wasser- und/oder Salzeinwirkung, Fäkalien, Chemikalien oder Lebensmitteln beständig sein sollen.

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende Beschichtung auf überwiegend ebene Flächen oder als Anstrich appliziert werden kann. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 2'000 mPa·s, bevorzugt im Bereich von 300 bis 1'500 mPa·s, insbesondere im Bereich von 300 bis 1'000 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt beispielsweise durch Aufgiessen auf das zu beschichtende Substrat. Dabei wird die Zusammensetzung im flüssigen Zustand mit Hilfe beispielsweise eines Rakels oder einer Zahntraufel gleichmässig verteilt. Zusätzlich kann die verteilte Zusammensetzung mit einer Stachelwalze egalisiert und entlüftet werden. Die Applikation kann aber auch manuell durch Pinsel oder Roller oder als Spritzapplikation erfolgen, beispielsweise als Korrosionsschutzbeschichtung auf Stahl. Bei der Aushärtung entstehen typischerweise weitgehend klare, glänzende und nichtklebrige Filme von hoher Härte und Beständigkeit und einer geringen Verfärbungsneigung, welche eine gute Haftung zu verschiedensten Substraten aufweisen. Mit den beschriebenen Härtern sind Epoxidharz-Beschichtungen zugänglich, welche auch unter ungünstigen, das heisst das Blushing begünstigenden, Reaktionsbedingungen, insbesondere bei tiefen Aushärtungstemperaturen im Bereich von 5 bis 10°C und hoher Luftfeuchtigkeit, zu Filmen von hoher Qualität aushärten.

Ein weiterer Gegenstand der Erfindung ist ein Artikel enthaltend eine ausgehärtete Zusammensetzung, erhalten durch die Aushärtung der beschriebenen Epoxidharz-Zusammensetzung. Die ausgehärtete Zusammensetzung liegt dabei insbesondere in Form einer Beschichtung vor.

Die beschriebene Epoxidharz-Zusammensetzung zeichnet sich durch vorteilhafte Eigenschaften aus. Sie weist nur wenig Geruch auf und ist auch ohne weitere Verdünner gut verarbeitbar. Sie ist überraschend niedrigviskos und härtet bei Umgebungstemperatur überraschend schnell und ohne störende Blushing-Effekte aus, insbesondere auch unter feucht-kalten Bedingungen. Im ausgehärteten Zustand weist sie hohe Härten und Beständigkeiten und eine geringe Verfärbungsneigung auf.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

### 1. Beschreibung der Messmethoden

Der **Amingehalt,** das heisst der totale Gehalt an Aminogruppen in den hergestellten Verbindungen, wurde titrimetrisch bestimmt (mit 0.1 N HClO₄ in Eisessig, gegen Kristallviolett) und ist stets angegeben in mmol N/g. **Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).
Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10-100 s⁻¹) gemessen.

### 2. verwendete Substanzen:

| | |
|---|---|
| TETA: | Triethylentetramin (techn., Amingehalt ca. 25.7 mmol N/g) |
| N4-Amin: | N,N'-Bis(3-aminopropyl)ethylendiamin (von BASF) |
| Jeffamine® D-230: | Polypropylenglykol-Diamin, mittleres Molekulargewicht ca. 240 g/mol, Amingehalt ca. 8.29 mmol N/g, (von Huntsman) |
| Jeffamine® T-403: | Polypropylenglykol-Triamin, mittleres Molekulargewicht ca. 460 g/mol, Amingehalt ca. 6.29 mmol N/g, (von Huntsman) |
| Araldite® GY 250: | Bisphenol-A-Diglycidylether; |
| | Epoxy-Equivalent ca. 187.5 g/Eq, (von Huntsman) |
| Epikote® 862 | Bisphenol-F-Diglycidylether; |
| | Epoxid-Equivalentgewicht ca. 169 g/Eq, (von Hexion) |
| Araldite® DY-E: | Monoglycidylether eines C₁₂- bis C₁₄-Alkohols; |
| | Epoxy-Equivalent ca. 290 g/Eq, (von Huntsman) |
| Ancamine® K 54: | 2,4,6-Tris-(dimethylaminomethyl)phenol (von Air Products) |

### 3. Herstellung von Härtern

### Allgemeine Herstellvorschrift für die reduktive Alkylierung

In einem Rundkolben wurden der Aldehyd und ein Amin unter Stickstoffatmosphäre in ausreichend Isopropanol gelöst. Die Lösung wurde während 30 Minuten bei Raumtemperatur gerührt und anschliessend bei einem WasserstoffDruck von 80 bar, einer Temperatur von 80 °C und einem Fluss von 3 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die Lösung im Vakuum bei 80 °C eingeengt.

**Härter *H1:*** Gemäss der allgemeinen Herstellvorschrift für die reduktive Alkylierung wurden 29.8 g 4-Dimethylaminobenzaldehyd und 18.4 g TETA umgesetzt. Erhalten wurde ein klares, leicht gelbliches Öl mit einer Viskosität von 750 mPa·s bei 20 °C und einem Amingehalt von 14.00 mmol N/g.
FT-IR: 2881, 2801, 1613, 1524, 1442, 1340, 1222, 1161, 1127, 946, 800, 760.

**Härter H2:** Gemäss der allgemeinen Herstellvorschrift für die reduktive Alkylierung wurden 29.8 g 4-Dimethylaminobenzaldehyd und 17.4 g N4-Amin umgesetzt. Erhalten wurde ein klares, leicht gelbliches Öl mit einer Viskosität von 870 mPa·s bei 20 °C und einem Amingehalt von 12.69 mmol N/g.
FT-IR: 2921, 2880, 2799, 1613, 1519, 1443, 1340, 1221, 1185, 1162, 1124, 1059, 946, 802, 749.

**Härter *H3***: Gemäss der allgemeinen Herstellvorschrift für die reduktive Alkylierung wurde eine Mischung aus 6.0 g 4-Dimethylaminobenzaldehyd und 17.0 g Benzaldehyd mit 17.4 g N4-Amin umgesetzt. Erhalten wurde ein klares, leicht gelbliches Öl mit einer Viskosität von 300 mPa·s bei 20 °C und einem Amingehalt von 10.94 mmol N/g.
FT-IR: 2924, 2882, 2805, 1614, 1521, 1494, 1452, 1340, 1162, 1117, 1073, 1060, 1027, 946, 804, 730.

**Härter H4:** Gemäss der allgemeinen Herstellvorschrift für die reduktive Alkylierung wurde eine Mischung aus 14.9 g 4-Dimethylaminobenzaldehyd, 8.5 g Benzaldehyd und 2.4 g Salicylaldehyd mit 17.4 g N4-Amin umgesetzt. Erhalten wurde ein klares, gelbliches Öl mit einer Viskosität von 530 mPa·s bei 20 °C und einem Amingehalt von 12.37 mmol N/g.
FT-IR: 2923, 2882, 2803, 1613, 1567, 1520, 1452, 1341, 1258, 1223, 1186, 1162, 1114, 1059, 1027, 946, 803, 750, 733, 698.

**Härter *H5***: Gemäss der allgemeinen Herstellvorschrift für die reduktive Alkylierung wurden 29.8 g 4-Dimethylaminobenzaldehyd und 11.6 g 1,5-Diamino-2-methylpentan umgesetzt. Erhalten wurde ein klares, gelbliches Öl mit einer Viskosität von 570 mPa·s bei 20 °C und einem Amingehalt von 10.15 mmol N/g.
FT-IR: 2921, 2870, 2846, 2798, 1613, 1567, 1519, 1475, 1442, 1340, 1223, 1183, 1162, 1127, 1110, 1059, 946, 801, 753.

**Härter *H6***: Gemäss der allgemeinen Herstellvorschrift für die reduktive Alkylierung wurden 29.8 g 4-Dimethylaminobenzaldehyd und 13.6 g 1,3-Bis-(aminomethyl)benzol umgesetzt. Erhalten wurde ein klares, leicht gelbliches Öl mit einer Viskosität von 510 mPa·s bei 20 °C und einem Amingehalt von 9.95 mmol N/g.
FT-IR: 2780, 1612, 1517, 1340, 1159, 945, 800.

**Härter *H7***: Gemäss der allgemeinen Herstellvorschrift für die reduktive Alkylierung wurden 29.8 g 4-Dimethylaminobenzaldehyd und 24.0 g Jeffamine® D-230 umgesetzt. Erhalten wurde ein klares, leicht gelbliches Öl mit einer Viskosität von 250 mPa·s bei 20 °C und einem Amingehalt von 7.45 mmol N/g.
FT-IR: 2966, 2864, 2800, 1614, 1521, 1444, 1340, 1224, 1161, 1102, 1062, 946, 804.

**Härter *H8:*** Gemäss der allgemeinen Herstellvorschrift für die reduktive Alkylierung wurden 20.3 g 4-Dimethylaminobenzaldehyd und 20.0 g Jeffamine® T-403 umgesetzt. Erhalten wurde ein klares, leicht gelbliches Öl mit einer Viskosität von 190 mPa·s bei 20 °C und einem Amingehalt von 6.61 mmol N/g.
FT-IR: 2965, 2865, 1615, 1522, 1445, 1373, 1342, 1161, 1102, 947, 804.

**Härter *HV1* (Vergleich):** Gemäss der allgemeinen Herstellvorschrift für die reduktive Alkylierung wurden 17.2 g Benzaldehyd und 15.0 g TETA umgesetzt. Erhalten wurde ein klares, leicht gelbliches Öl mit einer Viskosität von 260 mPa·s bei 20 °C und einem Amingehalt von 13.10 mmol N/g.

**Härter *HV2* (Vergleich):** Gemäss der allgemeinen Herstellvorschrift für die reduktive Alkylierung wurden 21.2 g Benzaldehyd und 11.6 g 1,5-Diamino-2-methylpentan umgesetzt. Erhalten wurde ein klares, leicht gelbliches Öl mit einer Viskosität von 420 mPa·s bei 20 °C und einem Amingehalt von 6.67 mmol N/g.

**Härter *HV3* (Vergleich):** Gemäss der allgemeinen Herstellvorschrift für die reduktive Alkylierung wurden 21.2 g Benzaldehyd und 13.6 g 1,3-Bis-(aminomethyl)benzol umgesetzt. Erhalten wurde ein klares, leicht gelbliches Öl mit einer Viskosität von 230 mPa·s bei 20 °C und einem Amingehalt von 6.41 mmol N/g.

### 4. Herstellung von Epoxidharz-Zusammensetzungen

Für jedes Beispiel wurden die in der Tabelle 1 angegebenen Inhaltsstoffe in den angegebenen Mengen (in Gewichtsteilen) mittels eines Zentrifugalmischers (SpeedMixer™ DAC 150, FlackTek Inc.) vermischt. 10 Minuten nach dem Vermischen wurde jeweils die Viskosität bei 20 °C der Zusammensetzungen bestimmt ("Viskosität (10')"). Weiterhin wurde jeweils ein erster Film in einer Schichtdicke von 500 µm auf eine Glasplatte aufgezogen und dieser bei 23±1 °C und 50±5 % relativer Feuchtigkeit (= Normklima, im folgenden abgekürzt mit "NK") gelagert, beziehungsweise ausgehärtet. An diesen Filmen wurde die Königshärte (Pendelhärte nach König, gemessen nach DIN EN ISO 1522) nach 2 Tagen ("Königshärte (NK) (2d)") bzw. nach 4 Tagen ("Königshärte (NK) (4d)") bzw. nach 7 Tagen ("Königshärte (NK) (7d)") bzw. nach 4 Wochen ("Königshärte (NK) (4w)") bestimmt. Nach 4 Wochen wurde der Aspekt der Filme beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet. Nach weiteren 4 Wochen im NK wurde die Farbe der Filme beurteilt. Weiterhin wurde jeweils ein zweiter Film in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 4 Wochen im NK gelagert, beziehungsweise ausgehärtet. Anschliessend wurde der Aspekt dieser Filme beurteilt (in den Tabellen mit "Aspekt (8°/80%)" bezeichnet), auf die gleiche Weise wie für den Aspekt (NK) beschrieben. An den so ausgehärteten Filmen wurde wiederum die Königshärte bestimmt, jeweils nach 7 Tagen bei 8 °C und 80 % relativer Feuchtigkeit ("Königsh. (8°/80%) (7d kalt)"), dann nach weiteren 2 Tagen im NK ("Königsh. (8°/80%) (+2d NK)") bzw. 7 Tagen im NK ("Königsh. (8°/80%) (+7d NK)") bzw. 4 Wochen im NK ("Königsh. (8°/80%) (+4w NK)").
Die Resultate sind in der Tabelle 1 angegeben.

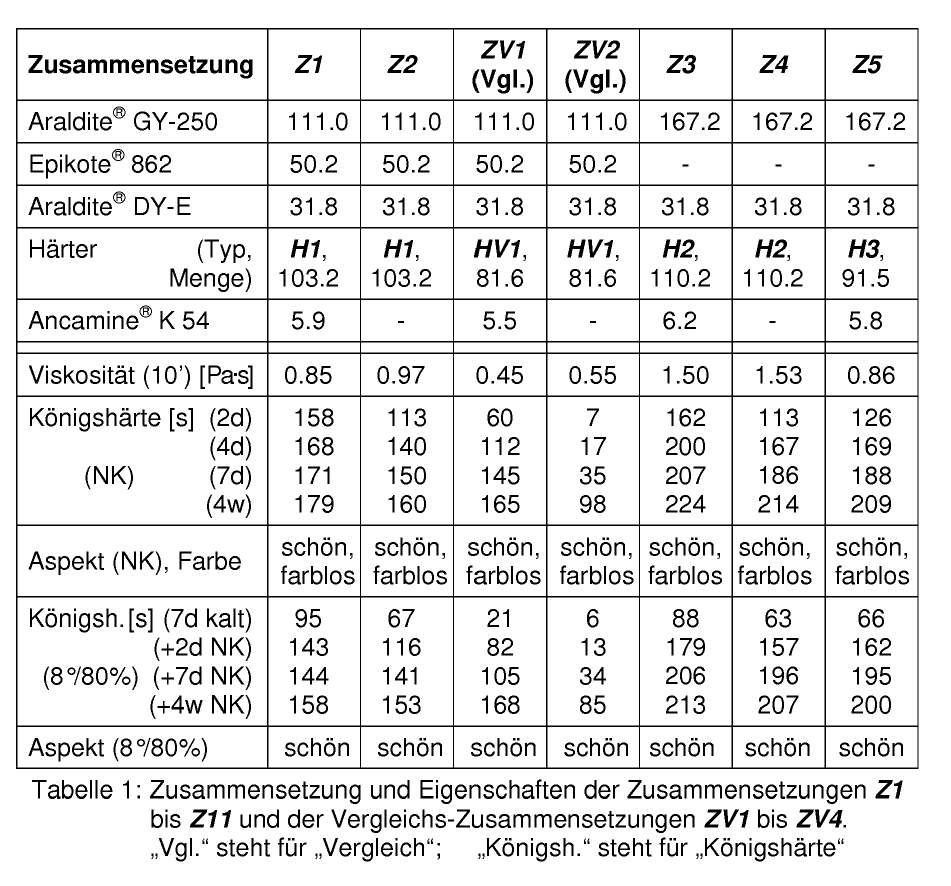

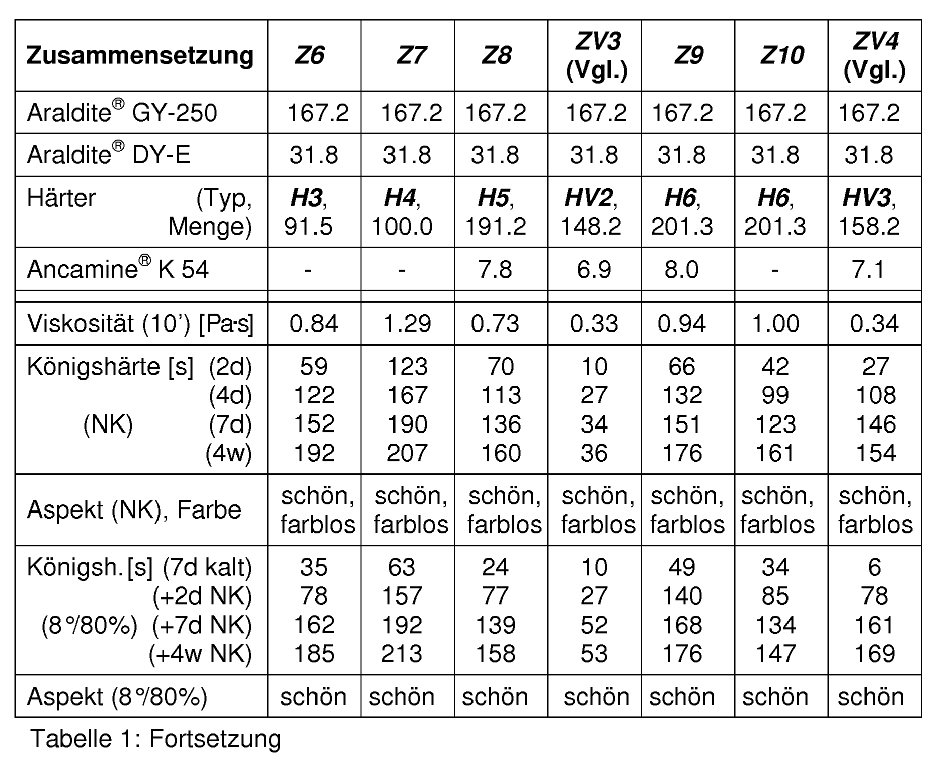

## Patentansprüche

1. Epoxidharz-Zusammensetzung enthaltend mindestens ein Epoxidharz und mindestens einen Härter, geeignet zum Aushärten von Epoxidharzen, enthaltend mindestens ein Amin mit mindestens einer Aminogruppe der Formel (I), 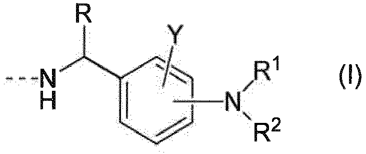 wobei
R für ein Wasserstoffatom oder eine Alkylgruppe mit 1 bis 12 C-Atomen steht;
R¹ und R² jeweils unabhängig voneinander für eine Alkyl- oder Arylalkylgruppe mit 1 bis 12C-Atomen stehen, oder zusammen für eine Alkylengruppe mit 4 bis 5C-Atomen stehen; und
Y für ein Wasserstoffatom oder für eine Alkyl- oder Alkoxygruppe mit 1 bis 12C-Atomen steht.

2. Epoxidharz-Zusammensetzung gemäss Anspruch 1, **dadurch gekennzeichnet, dass** das Amin mit mindestens einer Aminogruppe der Formel (I) eine, zwei oder drei Aminogruppen der Formel (I) aufweist.

3. Epoxidharz-Zusammensetzung gemäss einem der Ansprüche 1 oder 2, **dadurch gekennzeichnet, dass** R für ein Wasserstoffatom oder eine Methylgruppe steht.

4. Epoxidharz-Zusammensetzung gemäss einem der Ansprüche 1 bis 3, **dadurch gekennzeichnet, dass** R¹ und R² jeweils für eine Alkylgruppe mit 1 bis 4C-Atomen stehen.

5. Epoxidharz-Zusammensetzung gemäss einem der Ansprüche 1 bis 4, **dadurch gekennzeichnet, dass** Y für ein Wasserstoffatom oder für eine Methylgruppe steht.

6. Epoxidharz-Zusammensetzung gemäss einem der Ansprüche 1 bis 5, **dadurch gekennzeichnet, dass** Y für ein Wasserstoffatom und R¹ und R² je für eine Methylgruppe stehen und die tertiäre Aminogruppe in para-Stellung steht.

7. Epoxidharz-Zusammensetzung gemäss einem der Ansprüche 1 bis 6, **dadurch gekennzeichnet, dass** das Amin mit mindestens einer Aminogruppe der Formel (I)
entweder ein Amin der Formel (II) darstellt, 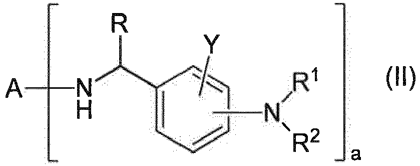 wobei
A für einen a-wertigen Kohlenwasserstoffrest mit einem Molekulargewicht im Bereich von 28 bis 5000 g/mol, welcher gegebenenfalls Ethergruppen, Aminogruppen, Hydroxylgruppen oder Mercaptogruppen aufweist, steht; und
a für eine ganze Zahl von 1 bis 3 steht;
oder ein Addukt eines Amins der Formel (II) mit mindestens einer Verbindung enthaltend mindestens eine Reaktivgruppe ausgewählt aus der Gruppe bestehend aus Epoxid-, Episulfid-, Aziridin-, Cyclocarbonat-, Isocyanat-, Isothiocyanat-, Acryl-, Methacryl- und Acrylamidgruppen darstellt.

8. Epoxidharz-Zusammensetzung gemäss Anspruch 7, **dadurch gekennzeichnet, dass** A entweder für einen a-wertigen Alkyl-, Cycloalkyl- oder Arylalkylrest mit 2 bis 20C-Atomen oder für einen a-wertigen Polyalkylenaminrest mit 1 bis 10 sekundären Aminogruppen oder für einen a-wertigen Polyoxyalkylenrest mit 1 bis 7 Ethergruppen steht,wobei diese Reste eine oder zwei primäre oder sekundäre aliphatische Aminogruppen aufweisen können.

9. Epoxidharz-Zusammensetzung gemäss einem der Ansprüche 7 oder 8, **dadurch gekennzeichnet, dass** das Addukt ein Addukt eines Amins der Formel (II) mit mindestens einem Mono- oder Polyepoxid ist.

10. Epoxidharz-Zusammensetzung gemäss einem der Ansprüche 7 oder 8, **dadurch gekennzeichnet, dass** das Amin mit mindestens einer Aminogruppe der Formel (I) ein Amin der Formel (II) darstellt.

11. Verwendung eines Härters gemäss Beschreibung in einem der Ansprüche 1 bis 10 zum Aushärten von mindestens einem Epoxidharz.

12. Ausgehärtete Zusammensetzung erhalten aus der Aushärtung der Epoxidharz-Zusammensetzung gemäss einem der Ansprüche 1 bis 10.

13. Artikel enthaltend eine ausgehärtete Zusammensetzung gemäss Anspruch 12.

14. Härter, geeignet zum Aushärten von Epoxidharzen, enthaltend mindestens ein Amin mit mindestens einer Aminogruppe der Formel (I), 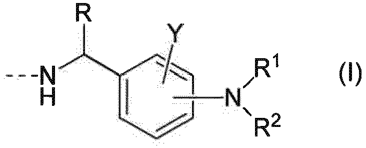 wobei
R für ein Wasserstoffatom oder eine Alkylgruppe mit 1 bis 12 C-Atomen steht;
R¹ und R² jeweils unabhängig voneinander für eine Alkyl- oder Arylalkylgruppe mit 1 bis 12C-Atomen stehen, oder zusammen für eine Alkylengruppe mit 4 bis 5C-Atomen stehen;
Y für ein Wasserstoffatom oder für eine Alkyl- oder Alkoxygruppe mit 1 bis 12C-Atomen steht; und
wobei das Amin mit mindestens einer Aminogruppe der Formel (I) nicht für ein Amin der Formel (VI) steht.

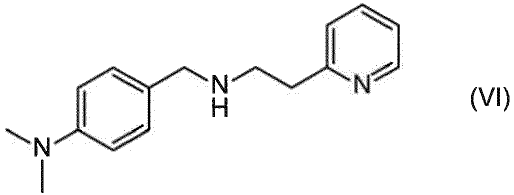

15. Härter gemäss Anspruch 14, **dadurch gekennzeichnet, dass** das Amin mit mindestens einer Aminogruppe der Formel (I)
entweder ein Amin der Formel (II) darstellt, 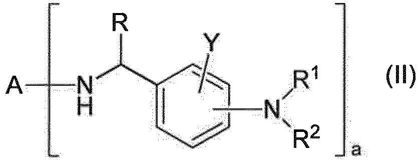 wobei
A für einen a-wertigen Kohlenwasserstoffrest mit einem Molekulargewicht im Bereich von 28 bis 5000 g/mol, welcher gegebenenfalls Ethergruppen, Aminogruppen, Hydroxylgruppen oder Mercaptogruppen aufweist, steht; und
a für eine ganze Zahl von 1 bis 3 steht;
oder ein Addukt eines Amins der Formel (II) mit mindestens einer Verbindung enthaltend mindestens eine Reaktivgruppe ausgewählt aus der Gruppe bestehend aus Epoxid-, Episulfid-, Aziridin-, Cyclocarbonat-, Isocyanat-, Isothiocyanat-, Acryl-, Methacryl- und Acrylamidgruppen darstellt.

## Claims

1. Epoxy resin composition comprising at least one epoxy resin and at least one hardener suitable for the curing of epoxy resins, comprising at least one amine having at least one amino group of the formula (I), 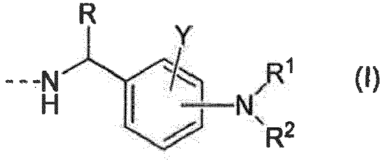 where
R is a hydrogen atom or an alkyl group having 1 to 12 carbon atoms;
R¹ and R² each independently of one another are an alkyl or arylalkyl group having 1 to 12 carbon atoms, or together are an alkylene group having 4 to 5 carbon atoms; and
Y is a hydrogen atom or is an alkyl or alkoxy group having 1 to 12 carbon atoms.

2. Epoxy resin composition according to Claim 1, **characterized in that** the amine having at least one amino group of the formula (I) has one, two or three amino groups of the formula (I).

3. Epoxy resin composition according to either of Claims 1 and 2, **characterized in that** R is a hydrogen atom or a methyl group.

4. Epoxy resin composition according to any of Claims 1 to 3, **characterized in that** R¹ and R² are each an alkyl group having 1 to 4 carbon atoms.

5. Epoxy resin composition according to any of Claims 1 to 4, **characterized in that** Y is a hydrogen atom or is a methyl group.

6. Epoxy resin composition according to any of Claims 1 to 5, **characterized in that** Y is a hydrogen atom and R¹ and R² are each a methyl group and the tertiary amino group is in para-position.

7. Epoxy resin composition according to any of Claims 1 to 6, **characterized in that** the amine having at least one amino group of the formula (I)
either is an amine of the formula (II), 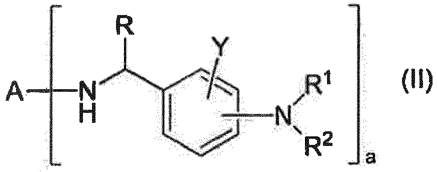 where
A is an a-valent hydrocarbyl radical having a molecular weight in the range from 28 to 5000 g/mol which optionally has ether groups, amino groups, hydroxyl groups or mercapto groups; and
a is an integer from 1 to 3;
or is an adduct of an amine of the formula (II) with at least one compound containing at least one reactive group selected from the group consisting of epoxide, episulphide, aziridine, cyclocarbonate, isocyanate, isothiocyanate, acryloyl, methacryloyl and acrylamide groups.

8. Epoxy resin composition according to Claim 7, **characterized in that** A alternatively is an a-valent alkyl, cycloalkyl or arylalkyl radical having 2 to 20 carbon atoms or is an a-valent polyalkylenamine radical having 1 to 10 secondary amino groups or is an a-valent polyoxyalkylene radical having 1 to 7 ether groups, it being possible for these radicals to have one or two primary or secondary aliphatic amino groups.

9. Epoxy resin composition according to either of Claims 7 and 8, **characterized in that** the adduct is an adduct of an amine of the formula (II) with at least one mono- or polyepoxide.

10. Epoxy resin composition according to either of Claims 7 and 8, **characterized in that** the amine having at least one amino group of the formula (I) is an amine of the formula (II).

11. Use of a hardener as described in any of Claims 1 to 10 for curing at least one epoxy resin.

12. Cured composition obtained from the curing of the epoxy resin composition according to any of Claims 1 to 10.

13. Article comprising a cured composition according to Claim 12.

14. Hardener suitable for curing epoxy resins, comprising at least one amine having at least one amino group of the formula (I), 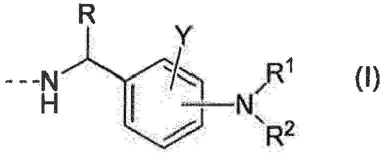 where
R is a hydrogen atom or an alkyl group having 1 to 12 carbon atoms;
R¹ and R² each independently of one another are an alkyl or arylalkyl group having 1 to 12 carbon atoms, or together are an alkylene group having 4 to 5 carbon atoms; and
Y is a hydrogen atom or is an alkyl or alkoxy group having 1 to 12 carbon atoms; and
where the amine having at least one amino group of the formula (I) is not an amine of the formula (VI).

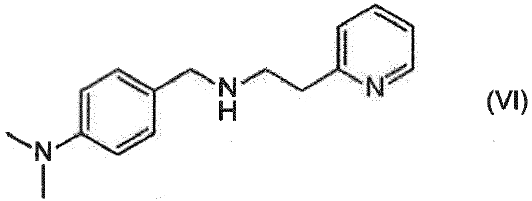

15. Hardener according to Claim 14, **characterized in that** the amine having at least one amino group of the formula (I)
either is an amine of the formula (II), 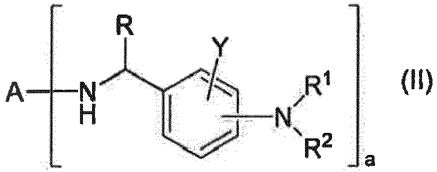 where
A is an a-valent hydrocarbyl radical having a molecular weight in the range from 28 to 5000 g/mol which optionally has ether groups, amino groups, hydroxyl groups or mercapto groups; and
a is an integer from 1 to 3;
or is an adduct of an amine of the formula (II) with at least one compound containing at least one reactive group selected from the group consisting of epoxide, episulphide, aziridine, cyclocarbonate, isocyanate, isothiocyanate, acryloyl, methacryloyl and acrylamide groups.

## Revendications

1. Composition de résine époxyde contenant au moins une résine époxyde et au moins un durcisseur, approprié pour le durcissement de résines époxydes, contenant au moins une amine comprenant au moins un groupe amino de formule (I) 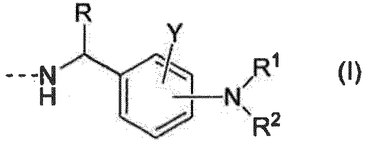 dans laquelle
R représente un atome d'hydrogène ou un groupe alkyle de 1 à 12 atomes C ;
R¹ et R² représentent chacun indépendamment l'un de l'autre un groupe alkyle ou arylalkyle de 1 à 12 atomes C, ou représentent ensemble un groupe alkylène de 4 à 5 atomes C ; et
Y représente un atome d'hydrogène ou un groupe alkyle ou alcoxy de 1 à 12 atomes C.

2. Composition de résine époxyde selon la revendication 1, **caractérisée en ce que** l'amine comprenant au moins un groupe amino de formule (I) comprend un, deux ou trois groupes amino de formule (I).

3. Composition de résine époxyde selon l'une quelconque des revendications 1 ou 2, **caractérisée en ce que** R représente un atome d'hydrogène ou un groupe méthyle.

4. Composition de résine époxyde selon l'une quelconque des revendications 1 à 3, **caractérisée en ce que** R¹ et R² représentent chacun un groupe alkyle de 1 à 4 atomes C.

5. Composition de résine époxyde selon l'une quelconque des revendications 1 à 4, **caractérisée en ce que** Y représente un atome d'hydrogène ou un groupe méthyle.

6. Composition de résine époxyde selon l'une quelconque des revendications 1 à 5, **caractérisée en ce que** Y représente un atome d'hydrogène et R¹ et R² représentent chacun un groupe méthyle, et le groupe amino tertiaire se trouve en position para.

7. Composition de résine époxyde selon l'une quelconque des revendications 1 à 6, **caractérisée en ce que** l'amine comprenant au moins un groupe amino de formule (I) est soit une amine de formule (II) 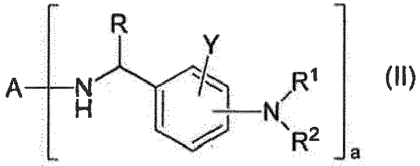 dans laquelle
A représente un radical hydrocarboné a-valent ayant un poids moléculaire dans la plage allant de 28 à 5 000 g/mol, qui comprend éventuellement des groupes éther, des groupes amino, des groupes hydroxyle ou des groupes mercapto ; et
a représente un nombre entier de 1 à 3 ;
soit un adduit d'une amine de formule (II) avec au moins un composé contenant au moins un groupe réactif choisi dans le groupe constitué par les groupes époxyde, épisulfure, aziridine, cyclocarbonate, isocyanate, isothiocyanate, acryle, méthacryle et acrylamide.

8. Composition de résine époxyde selon la revendication 7, **caractérisée en ce qu'**A représente un radical alkyle, cycloalkyle ou arylalkyle a-valent de 2 à 20 atomes C ou un radical polyalkylène-amine a-valent contenant 1 à 10 groupes amino secondaires ou un radical polyoxyalkylène a-valent contenant 1 à 7 groupes éther, ces radicaux pouvant comprendre un ou deux groupes amino aliphatiques primaires ou secondaires.

9. Composition de résine époxyde selon l'une quelconque des revendications 7 ou 8, **caractérisée en ce que** l'adduit est un adduit d'une amine de formule (II) avec au moins un mono- ou polyépoxyde.

10. Composition de résine époxyde selon l'une quelconque des revendications 7 ou 8, **caractérisée en ce que** l'amine comprenant au moins un groupe amino de formule (I) est une amine de formule (II).

11. Utilisation d'un durcisseur selon la description dans l'une quelconque des revendications 1 à 10 pour le durcissement d'au moins une résine époxyde.

12. Composition durcie obtenue par le durcissement de la composition de résine époxyde selon l'une quelconque des revendications 1 à 10.

13. Article contenant une composition durcie selon la revendication 12.

14. Durcisseur, approprié pour le durcissement de résines époxydes, contenant au moins une amine comprenant au moins un groupe amino de formule (I) 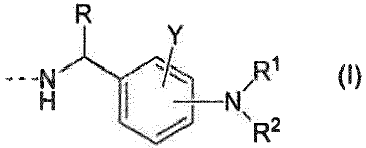 dans laquelle
R représente un atome d'hydrogène ou un groupe alkyle de 1 à 12 atomes C ;
R¹ et R² représentent chacun indépendamment l'un de l'autre un groupe alkyle ou arylalkyle de 1 à 12 atomes C, ou représentent ensemble un groupe alkylène de 4 à 5 atomes C ;
Y représente un atome d'hydrogène ou un groupe alkyle ou alcoxy de 1 à 12 atomes C ;
l'amine comprenant au moins un groupe amino de formule (I) n'étant pas une amine de formule (VI)

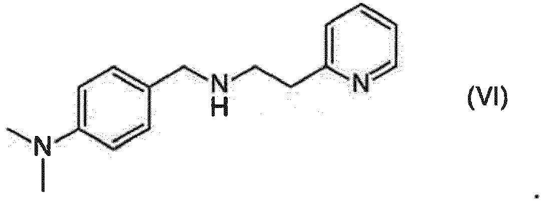

15. Durcisseur selon la revendication 14, **caractérisé en ce que** l'amine comprenant au moins un groupe amino de formule (I) est soit une amine de formule (II) 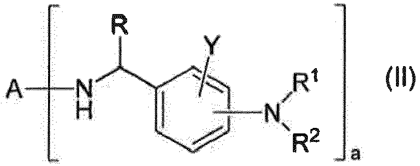 dans laquelle
A représente un radical hydrocarboné a-valent ayant un poids moléculaire dans la plage allant de 28 à 5 000 g/mol, qui comprend éventuellement des groupes éther, des groupes amino, des groupes hydroxyle ou des groupes mercapto ; et
a représente un nombre entier de 1 à 3 ;
soit un adduit d'une amine de formule (II) avec au moins un composé contenant au moins un groupe réactif choisi dans le groupe constitué par les groupes époxyde, épisulfure, aziridine, cyclocarbonate, isocyanate, isothiocyanate, acryle, méthacryle et acrylamide.
